# EUROPEAN PATENT SPECIFICATION

(11) **EP 1 906 195 B1**
(45) Date of publication and mention of the grant of the patent: **24.12.2008**
(21) Application number: 06121574.5
(22) Date of filing: 29.09.2006
(51) Int. Cl.: G01R 33/38

(54) **MRI apparatus and MRI method using such apparatus**
Gerät der Magnetresonanzbildgebung und Verfahren der Magnetresonanzbildgebung unter Verwendung eines derartigen Geräts
Dispositif d'IRM et procédé d'IRM utilisant un tel dispositif

(43) Date of publication of application: 02.04.2008
(60) Divisional application: 08160660.0
(73) Proprietor: Esaote S.p.A., 20100 Milano (IT)
(72) Inventor: Biglieri, Eugenio, I-15024, Masio (Al) (IT); Satragno, Luigi, I-16122, Genova (IT)
(74) Representative: Karaghiosoff, Giorgio Alessandro

(56) References cited:
- EP-A- 0 424 808
- EP-A- 0 984 293
- EP-A1- 0 753 758
- EP-A2- 0 121 367
- EP-A2- 0 931 505
- WO-A-20/05076026
- WO-A-20/05096927
- WO-A-20/05124380
- JP-A- 2 200 243
- JP-A- 3 106 335
- JP-A- 6 181 907
- JP-A- 9 131 332
- JP-A- 2006 136 388
- US-A- 4 920 318
- US-A- 5 680 861
- US-A- 5 850 143
- US-A- 5 905 378
- US-B1- 6 414 490
- US-B1- 6 617 852
- US-B1- 6 828 792
- US-B1- 6 934 574
- J.R.JINKINS ET AL.: "Upright, Weight-bearing, Dynamic-kinetic Magnetic Resonance Imaging of the Spine - Review of the First Clinical Results" J. HK COLL. RADIOL., vol. 6, 2003, pages 55-74, XP002430581
- C.M.POWERS ET AL.: "Patellofemoral Kinematics During Weight-Bearing and Non-Weight-Bearing Knee Extension in Persons With Lateral Subluxation of the Patella: A Preliminary Study" JOURNAL OF ORTHOPAEDIC & SPORTS PHYSICAL THERAPY, vol. 33, no. 11, 2003, pages 677-685, XP002430582
- PEARLE A D ET AL: "JOINT MOTION IN AN OPEN MR UNIT USING MR TRACKING" JOURNAL OF MAGNETIC RESONANCE IMAGING, SOCIETY FOR MAGNETIC RESONANCE IMAGING, OAK BROOK, IL,, US, vol. 10, no. 1, July 1999 (1999-07), pages 8-14, XP008018579 ISSN: 1053-1807
- C.P.CHENG ET AL.: "Dynamic Exercise Imaging With an MR-Compatible Stationary Cycle Within the General Electric Open Magnet" MAGN.RESON.MED., vol. 49, 2003, pages 581-585, XP002430583
- J.M.SANTOS ET AL.: "Full-Flexion Patellofemoral Joint Kinematics with Real-Time MRI at 0.5T" PROC.INTL.SOC.MAG.RESON.MED. 13, 2005, page 1951, XP002430584
- P.WENDLING: "Weight-bearing MRI brings pathologies to light" FIND ARTICLES, [Online] 15 February 2005 (2005-02-15), XP002430585 Retrieved from the Internet: URL:http://findarticles.com/p/articles/mi_ m0CYD/is_4_40/ai_n12416143> [retrieved on 2007-04-20]
- D.WEISHAUPT ET AL.: "MR Imaging of the Forefoot under Weight-Bearing Conditions: Position-Related Changes of the Neurovascular Bundles and the Metatarsal Heads in Asymptomatic Volunteers" JMRI, vol. 16, 2002, pages 75-84, XP002430586
- K.MEETZ ET AL.: "Tracking of Anatomical Structures in 4D Kinematic MRI" PROC.INTL.SOC.MAG.RESON.MED. 13, 2005, page 1957, XP002430587
- J.F.ROY ET AL.: "A Study of the Morphology of Lumbar Discs in Sitting and Standing Positions Using a 0.5T Open-Configuration MRI" PROC.INTL.SOC.MAG.RESON.MED. 9, 2001, page 245, XP002430717
- R.COOPERSTEIN: "American Back Society Meets in San Francisco, Part II" DYNAMIC CHIROPRACTIC, [Online] vol. 16, 1998, XP002430718 Retrieved from the Internet: URL:http://www.chiroweb.com/archives/16/07 /19.html> [retrieved on 2007-04-20]

## Description

This invention relates to a MRI apparatus comprising a magnet structure which delimits a cavity in which a body under examination or a part thereof is received, and which includes means for generating a magnetic field in said cavity, as well as means for causing the body under examination or the part thereof to emit nuclear magnetic resonance signals and means for receiving said nuclear magnetic resonance signals which are electrically connected to the MRI apparatus.

MRI apparatus typically comprise a magnet structure, which structure is composed of a plurality of wall elements and/or columns or other load bearing elements, which are connected together to delimit a cavity for receiving the body of a patient or at least a part thereof, and at least one or a plurality of openings for said body and/or part thereof to access said cavity. Furthermore, the magnet structure also has means for generating a magnetic field permeating the cavity or a portion of the whole volume of said cavity, which means may include permanent magnets, resistive magnets or superconducting magnets or combinations of such magnets. These magnetic field generating means are supported and/or wholly or partly form the wall elements or the construction elements of the magnet structure.

In addition to the above magnet structure, having the function to delimit the cavity and generating the magnetic field, the MRI apparatus generally comprises so-called gradient coils for generating variable magnetic fields with selection and encoding functions, allowing to reconstruct the spatial relationship between resonance signals and the topological position of the source. Finally, required components include the transmitting coil, whereby a RF exciting pulse is generated, and the receiving coil which picks up the RF magnetic resonance signals emitted from the body under examination or the part thereof being imaged. Further auxiliary equipment or devices and/or units are intended as prior art, which help to improve or control parameters such as electromagnetic and magnetic noise, thermal drifts, electronic drifts, etc.

In the specific field of MR imaging, which is a widely used term in the art to define Magnetic Resonance imaging, two different families of apparatus are known, i.e. Total Body and Dedicated apparatus. The former have large magnet structures that generate large magnetic fields and their patient receiving cavities, as well as the accesses to such cavities have such a size as to allow the whole body to be received therein. Conversely, dedicated apparatus have magnet structures adapted to generate relatively low-strength magnetic fields, but both the structure and the cavity have a small size, so that only limited anatomical regions or limbs may be imaged therein, such as a lower or upper limb, a shoulder and/or a head.

Magnetic resonance imaging is increasingly used in medicine for orthopedic diagnostics. A critical factor in orthopedic diseases, in addition to the morphological condition of bone tissues, is functional dynamic behavior, especially in different stress conditions.

Therefore, it is important for the patient to be allowed to assume several different postures and be subjected to mechanical stresses as close as possible to natural conditions.

In certain prior art apparatus, various kinds of examinations may be performed with the patient in various positions and stress conditions, which mostly only simulate natural postures and stresses. In these apparatus, the devices for allowing the patient to assume the different postures and the best position in the cavity, as well as the devices required for resonance signal acquisition are different in terms of appearance and structure from the devices used by patients in their daily life, and generate psychological stress, fear and anxiety, causing muscular contractions which may at worst affect natural postures and generate restlessness conditions in the patient, thereby causing difficulties for the latter to keep still for the required examination time.

The invention mainly addresses the problem of providing a MRI apparatus that allows easy examination of several different anatomic regions under natural load or stress conditions, without requiring highly technically complex and expensive accessories and/or constructions and without inducing psychological stress, i.e. anxiety or fear, in patients.

Besides providing a magnet structure having a patient receiving space with access openings of such a size as to allow the patient to enter and exit by simply walking into and out of said patient receiving space through said openings, the invention advantageously aims to provide resonance signal receiving means that can be removably attached directly to the patient body, at one or more separate anatomic regions to be imaged.

Document WO2005/124380 discloses a clothing to which a plurality of radio frequency coils are attached, which are relatively movable with respect to one another.

US patent 5 905 378 discloses a surface coil system for use with an MR imaging system, the surface coil system consisting of a plurality of flexible coils removably fastened to a wearable support element, e.g. by means for Velcro-type attachments.

JP-A-02200243 discloses a modular surface coil system containing two blocks carrying an antenna conductor. Said blocks may be interconnected by a second block changing the size of the coil.

The invention achieves the above mentioned aims by means of an MRI apparatus according to claim 1.

The invention provides arrangements for adapting wearable receiving coils to a certain range of patient body sizes without affecting signal quality, signal power and/or signal-to-noise ratio, as well as without causing pain to patients or restricting conditions which might generate psychological stress or anxiety.

By placing the receiving coil closer to the signal source, a better signal-to-noise ratio is obtained, or at least the quality standards for such ratio may be kept constant in spite of a limitation of the static field strength and of excitation pulses. The shorter distance between the receiving coil and the relevant anatomic region compensates for the weaker resonance signals emitted by a relatively weak magnetic field. Therefore, using the construction parameters of total body magnets, the signal may be improved.

The receiving coil construction which is defined in claim 1 gives the patient the required freedom of movement to assume the various postures. Furthermore, the use of removable fastener means for attaching the receiving coil conductors to a band, strap or vest allows to optimize the coil shape and/or the path of the conductors that form it with respect to the relevant anatomic region and the patient's morphology.

The apparatus according to the invention provides maximum simplicity and flexibility as well as a modular design, which allows to reduce the number of required parts. These features allow to create several different shapes of antennas or receiving coils, by simple integration of base modules.

It will be appreciated that the invention always allows patient to access the apparatus by autonomous walking or other autonomous action even using auxiliary locomotion means, such as a manually or motor driven wheelchair. The apparatus of the invention further allows patient access using means pushed by third parties, such as a patient table, a convertible armchair or the like, e.g. as disclosed in patent EP 913,122 or US 6,346,814.

It may be further envisaged that the patient accesses the patient receiving space by simply walking therein and that a patient table, an armchair, a chair or another element for supporting the patient in a predetermined posture and/or a predetermined position relative to the magnetic structure is introduced or present therein, and the patient autonomously sits or lies thereon. The patient table, armchair, chair or any other support element may be arranged to move on wheels or skids and/or to be locked in one or more predetermined positions within the patient receiving space of the magnet structure thanks to means for stopping motion in the proper position, such as adjustable stops or abutments and/or means for indication of proper positioning allowing to stop the motion of the above patient supporting means.

The following detailed description and the dependent claim describe certain particular embodiments of the present method.

As mentioned above, the dependent claim relates to improvements of this invention.

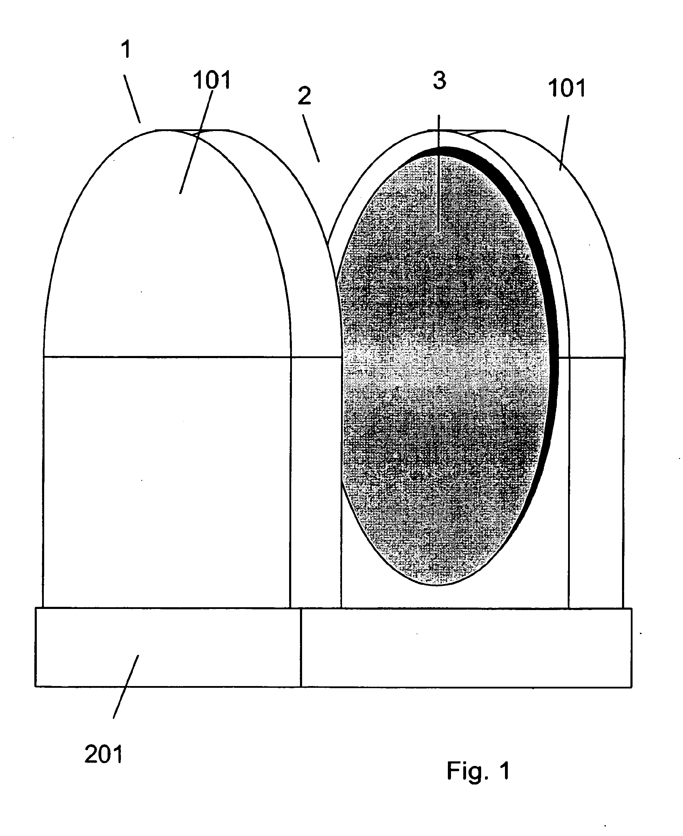

The characteristics of the invention and the advantages derived therefrom will appear more clearly from the following description of a few non limiting embodiments, illustrated in the annexed drawings, in which:
Fig. 1 is a perspective view of a magnet structure.
Fig. 2 is a view showing the channel between the two opposite pole pieces of the magnet structure in the direction of the longitudinal axis, which channel forms the patient receiving space and in which the patient is shown in a possible examination position.
Figs. 3 to 7 are sectional views taken along a vertical center plane parallel to the surfaces of the pole pieces of the magnet structure as shown in the previous figures and, in each of which Figures 3 to 7 the patient is shown in various postures, obtained thanks to one or more means for positioning and/or bearing and/or retaining and/or supporting the patient or for assisting placement thereof.
Fig. 8 is a diagrammatic view of an exemplary wearable coil, in the form of a band to be fastened around a part of the patient body.
Fig. 9 is a diagrammatic view of an exemplary construction of a receiving coil having flexible and extensible conductors, which are supported by a garment by means for removable attachment of conductors to such garment.
Figs. 10 and 11 are schematic views of different exemplary garment-conductor combinations, using means for removable attachment of conductors.
Figure 12 shows, like Figure 2, a patient standing in the patient receiving space of the magnet structure of the present apparatus, which patient wears a gravity ballast garment.
The arrangements shown in figures 1-7, 11 and 12, and described in the following, do not represent embodiments of the invention.
Particularly referring to Figure 1, a MRI apparatus generally comprises a magnet structure 1 which delimits a cavity 2 in or through which a body under examination or a part thereof P is received, and which includes means 3 for generating a magnetic field in said cavity, as well as means for causing the body under examination or the part thereof P to emit nuclear magnetic resonance signals and means for receiving said nuclear magnetic resonance signals.

The construction of magnetic resonance imaging apparatus has been long known. The magnet structure has the function of delimiting a space for receiving a patient or a part of the patient's body and of generating a magnetic field which permeates at least partly the volume of the patient receiving space. The magnet structure is also associated to so-called gradient coils for generating time dependent magnetic fields as well as coils for transmitting matter excitation pulses, which are generally radio frequency pulses, as well as at least one coil for receiving the radio frequency signals resulting from matter response to the excitation pulses.

A general introduction to the principles of operation of MRI apparatus is contained in: "Quaderni di RM, Basi Fisiche della Risonanza Magnetica" A. Desgrez, J.Bittoun, I.Idy-Peretti, Edizione Franco Milani 1992 Masson.

The means for generating the static field that permeates at least partly the patient receiving space may include permanent magnets and/or resistive magnets and/or superconducting magnets.

The type of field generating means has a minor incidence upon the operation principle of MRI apparatus. Construction variants are mainly associated to the means for controlling the resistive or superconducting magnets which require power in coils combined with cores and the corresponding drivers and power supplies. Temperature control is generally always provided even in permanent magnets, however in superconducting magnets in addition to reducing or compensating for thermal drifts of magnetic fields, this control also allows to maintain the conditions in which the coils have a superconducting behavior.

The MRI apparatus has many more components in addition to the magnet structure and the above mentioned operating units, however these components are known parts, which are not related to the present concepts.

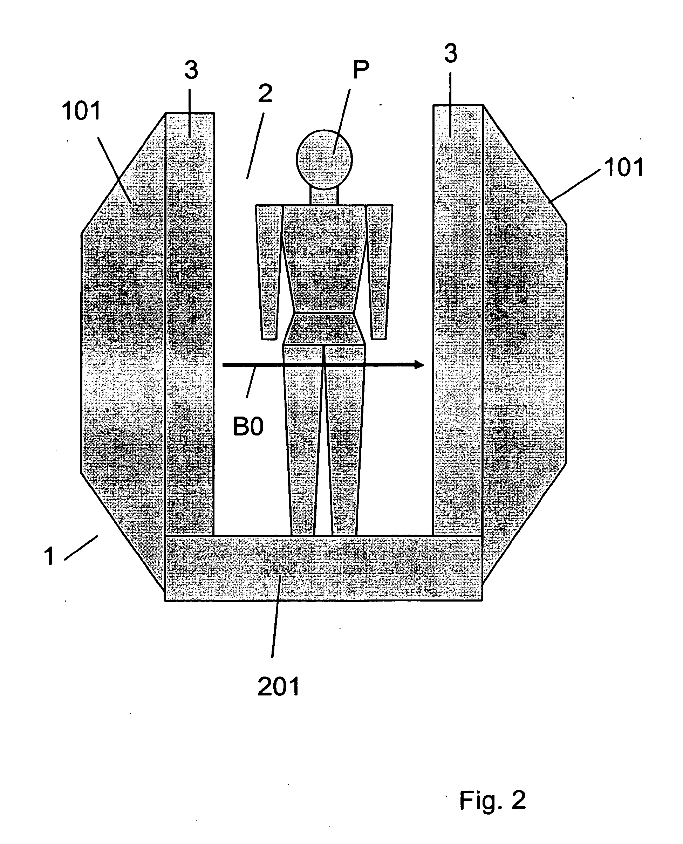

As shown in Figures 1 and 2, the magnet structure has two opposite pole pieces 101 delimiting a cavity whose shape provides at least one access opening through which patients can access said cavity by simply walking therein. Particularly, in this example, the cavity has the shape of a passage for connection of two opposite openings on two opposite vertical sides. The latter may also be parallel or substantially parallel to each other and to the direction of the magnetic field between the pole pieces 101.

Regarding the size, the cavity and the at least one access opening have such a width as to allow the patient to access it by walking and/or remain in such cavity in a position in which the axis that joins his/her shoulders is parallel to the width of the cavity.

In particular conditions, the cavity and the at least one access opening, particularly the two opposite access openings have such a width as to allow access by a wheelchair or similar device, either driven by the person sitting thereon or pushed by third parties.

The patient may be also introduced in the cavity 2 using a patient table, a convertible armchair and/or an armchair or the like, instead of the wheelchair, after laying the patient thereon outside the magnet structure. The patient table, armchair and/or other similar devices may also have means for adjusting the height and/or tilt of the patient support plane about one or more axes so that the patient may be tilted with different orientations in space.

The two opposite field generating pole pieces form or are supported by two opposite vertical walls which are spaced to such an extent as to form the above mentioned patient receiving space and at least one access opening at least at one end side. The means for excitation of the body under examination or a part thereof for emission of resonance signals (not shown in detail and generally consisting of at least one transmitting coil) are integrated in or supported by said walls or are combined with said pole pieces 101.

In this example, the wall that connects the two pole pieces 101 and/or the walls supporting such two pole pieces 101 is the support base for the magnet structure and the walking surface for the patient when he/she is within the patient receiving space.

A number of variants of the magnet structure may be provided, such as different rotations of the structure as shown in Figures 1 and 2, in which the wall 201 is in an upright position, wherefore the magnet structure is composed of three closed vertical walls, formed by the pole pieces 101 or the associated support walls, and the wall 201, and the walking surface is the floor itself or another base.

The base 201 that, in the specific non-limiting example of the figures, acts as a connection wall between the two opposite walls that form or support the pole pieces 101 of the magnet structure, may have one, two or more steps on the opening side, for access to the imaging space. Otherwise, to allow access by disabled people and/or by carriage-mounted means of transport, such as patient tables, armchairs or the like, there are provided at least two parallel climbing ramps at the at least one access opening, along parallel strips disposed at the same distance from each other as the wheels of a wheelchair, a patient table, an armchair, or the like, or one climbing ramp whose width corresponds to the total width of the base.

A balustrade and/or lateral gripping and retainer means may be further provided at least in the area of the steps and/or ramps and at least on one side or on both sides thereof, for assisting the patient in sliding into the space 2.

While Figure 2 shows a standing patient, with the axis that joins his/her two shoulders substantially perpendicular to the pole pieces and/or parallel to the magnetic field therebetween, the patient may willingly take any position or posture in the imaging space. For assisting the patient in assuming certain postures, which pose particular problems in terms of balance and/or physical effort, considering that imaging sequences of a MRI apparatus may be relatively long, the MRI apparatus may comprise means for positioning and/or bearing and/or retaining and/or supporting the patient or for assisting placement thereof. These are advantageously mountable, removable or even displaceable, separately or together, to and from the imaging space, thanks to their possibly motor-driven carriage-mounted design. Any type of particular and specific positioning and/or bearing and/or retaining and/or supporting means may be used, provided that the patient is not forced to assume any position, and that he/she can assume it by a wholly voluntary action, and preferably by a natural action or movement. This description only mentions a few examples, which are to be intended without limitation, although they cover the most typical and/or useful patient postures, especially for morphological and functional imaging.

Furthermore, while Figure 2 shows the standing patient P with the axis that joins his/her shoulders parallel or substantially parallel to the static field B0 generated between the pole pieces, the patient may assume any position with any inclination of the axis that joins his/her shoulders with respect to the magnetic field direction. Also, different orientations may be provided for different body parts, thence the patient may assume a posture in which the shoulders and the pelvis have different orientations, by rotating his/her torso, or other similar postures.

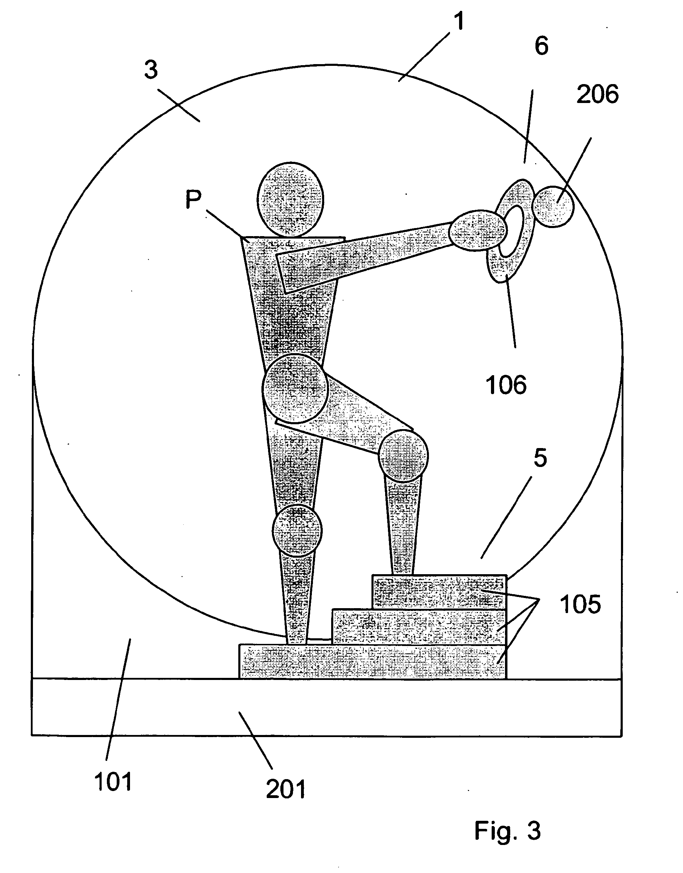

In a first example, as shown in Figure 3, the positioning means consist of one or more platforms 5 having different heights and an access side composed of one or more successive steps. These platforms 5 may be removably mounted in several different positions to the base 201 or the walking surface of the magnet structure within the patient receiving space. The platforms may also have a carriage-like support base, allowing displacement within the patient receiving space 2, removal therefrom and placement in a predetermined position. Braking means and/or stopping means, such as abutments, removable and/or adjustable limit stops, may allow the platform 5 to be temporarily locked within the patient receiving space 5 in one or more predetermined positions corresponding to one or more predetermined anatomic region- and/or organ-specific imaging positions. The static field B0 normally exhibits optimal homogeneity in a space volume extending over a portion of the total volume of the patient receiving space 2, which is generally approximately centered with respect to such total volume. Therefore, diagnostic imaging of an anatomic region or organ requires such region or organ to be placed in the partial volume in which the best magnetic field homogeneity can be achieved. This partial volume is known in the art as imaging volume.

According to an improvement, the platform has a modular construction, i.e. is composed of multiple platform elements 105, each having a predetermined height, e.g. a minimum unit height. Also, one or more platform elements may be diminished or increased in height, allowing height adjustment in a range from a minimum value to a maximum value. By this arrangement, coarse patient position adjustment with respect to the imaging volume, for the above purpose of centering the organ and/or anatomic region with the imaging volume, is performed using the modular elements, whereas fine position adjustment occurs thanks to the height or thickness adjustable element. In a variant thereof, this element is provided as a common base element which has additional carriage characteristics, so that only one module of the set is adjustable in thickness or height, whereas all the others are stationary, hence simpler and less expensive.

The platform elements 105 may be mounted in superimposed arrangements for making platforms 5 of various heights. They may be also arranged in laterally staggered position with respect to vertical alignment, to form a stepped access side.

A further example of the means for positioning and/or bearing and/or retaining and/or supporting the patient or for assisting placement thereof, that may be provided separately or in combination with other positioning means consists of one or more gripping handles 6 or other gripping means, which may be either permanently or removably fixed in several different positions with respect to the walls that form and/or support the pole pieces 101 and/or with respect to the base 201 and/or with respect to the platform 5 and to other means for positioning and/or bearing and/or retaining and/or supporting the patient or for assisting placement thereof, such as those that will be described in the following embodiments.

In Figure 3, the handles 6 are provided in combination with the platform 5 in such a position as to allow the patient to hang thereto with little effort in a position in which the knee and/or other musculoskeletal regions or other articulations are under stress, such as climbing a step. The handles 6 or any other different gripping means are permanently mounted to the magnet structure. Possibly, at least some of the gripping means, combined together, are arranged in multiple fixed positions, whereas at least some of the rest of such handles or gripping means are or may be mounted removably and/or adjustably in terms of position and orientation, and may assume several different positions with respect to the extension of the walls that support or form the pole pieces and/or the base and/or the platform/s.

In Figure 3, the handle 6 comprises a gripping element 106 which is fixed to the magnet structure by means of an upper horizontal beam 206. The gripping element may be secured to the beam by means of skids, which slide along the axis of the beam 206 and may be locked in position. Furthermore, the gripping element may be connected to the skids (not shown) by means of tilt or extension joints. Alternatively or in addition, the walls 101 of the magnet structure may have adjustable supports in one or more predetermined end positions of the beam 206 or two or more different fixed supports in different positions.

A great number of variants for fixation of the gripping means 106 may be envisaged, including any type of brackets or support elements, such as rods, posts and/or beams, each of which may be removably fixed in one or more predetermined positions to the base and/or the walls or the pole pieces of the magnet structure and/or the platform/s or the modular platform elements.

Referring to Figure 3, the gripping or retaining means 6 consist of at least one handle, preferably a pair of laterally aligned or misaligned handles 6, which are spaced in such a position as to provide support for forward bending of the standing patient body P, i.e. in a prone position thereof. In this case, the gripping element/s 106 of each handle are mounted to a vertical post 206. This post may be provided in several different lengths or be continuously adjustable in length.

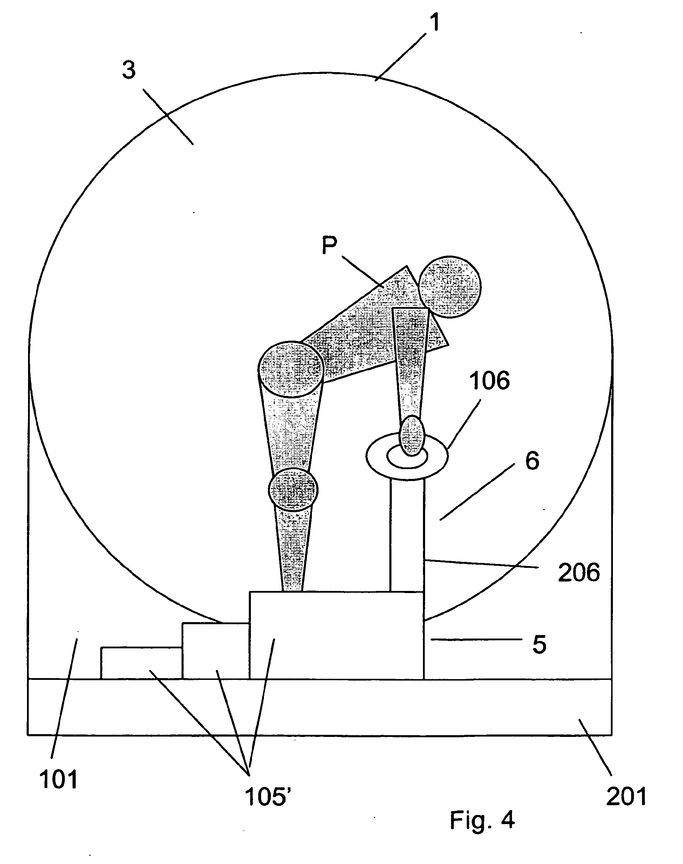

As shown in Figure 4, the handle 6 is provided in combination with a stepped base 5, which is composed of modules 105' having a different design from those described above, as an example of the many different alternative constructions that may be provided therefor. The post/s 206 for the handles 6 may be fixed to one of such modules 105' of the base, preferably in a removable manner.

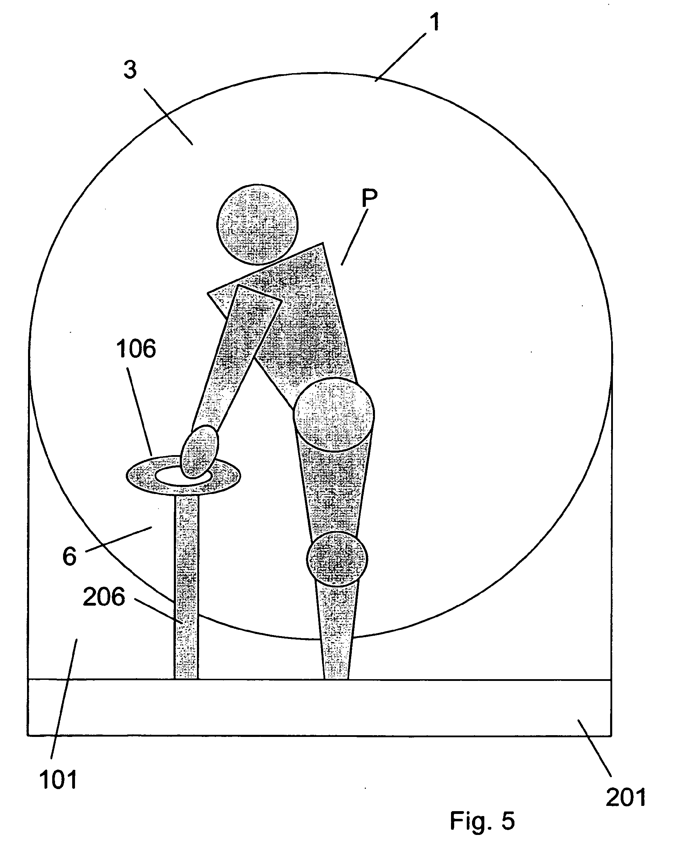

Figure 5 shows a variant handle positioning assembly, providing at least one handle, preferably a pair of laterally aligned or misaligned handles, which are spaced in such positions as to act as a support for the patient. In the specific condition of the figure, the patient is in a backwardly arched position, said handles being adjusted and positioned to act as a support for backward arching of the patient body. Nevertheless, these handles may be also used for forward arching when suitably adjusted and positioned in the patient receiving space 2.

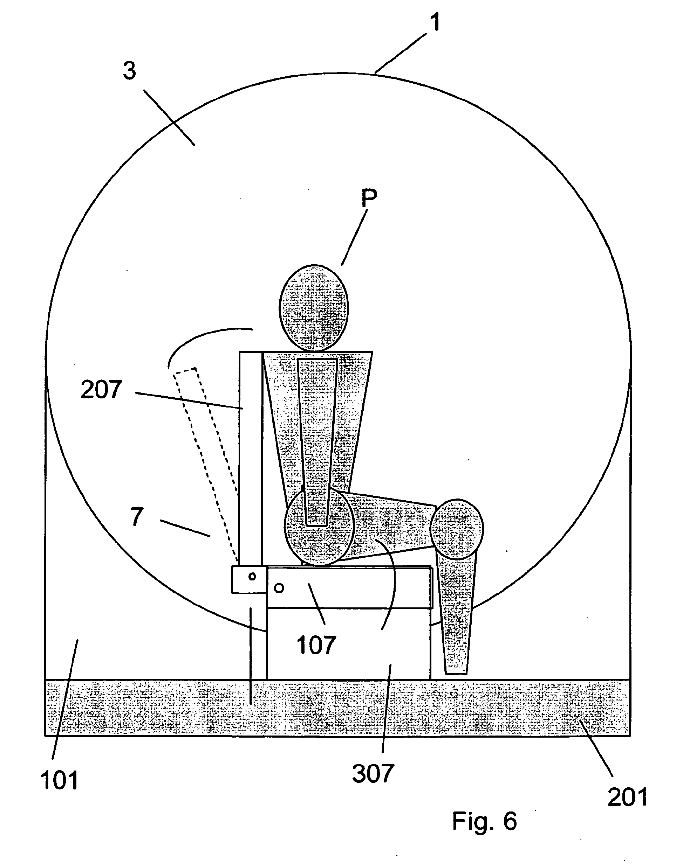

Figure 6 shows a further variant of the means for positioning and/or bearing and/or retaining and/or supporting the patient or for assisting placement thereof, which consist of at least one seat element 7 that may be removably mounted or positioned in different positions within the imaging space and on the base thereof.

The seat element may be provided in combination with one or more of the means 5 or 6 for positioning and/or bearing and/or retaining and/or supporting the patient or for assisting placement thereof may be provided as described above. Like the bases 5, the base 307 of the seat element may have a carriage-mounted design and/or be provided with means for adjusting the seat height position and/or means for tilt adjustment along one or more different axes of the seating surface 107 and/or the backrest 207. The seat 7 has at least one seating surface part 107 which is allowed to tilt along at least one axis, preferably along two perpendicular axes, and especially along at least one transverse axis, i.e. oriented in the seat width and/or magnetic field B0 direction (see Fig. 2). The seat 7 has at least one backrest part 207 which is allowed to tilt along at least one axis, preferably along two perpendicular axes, and especially along at least one transverse axis, i.e. oriented in the backrest width and/or magnetic field B0 direction (see Fig. 2).

The seat 7 may be also provided in combination with footrest elements (not shown) that may be removable and/or adjustable in tilt along at least one transverse axis, i.e. oriented in the backrest width direction, and/or along two perpendicular axes and/or possibly additionally or alternatively adjustable in height.

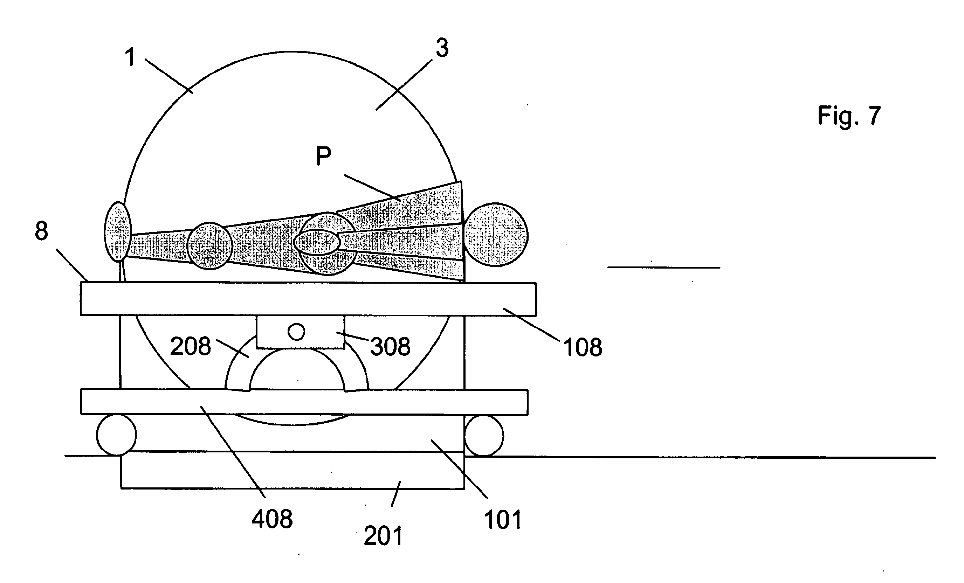

Referring to Figure 7, another positioning means may be a patient table 8. In its simplest form, the patient table 8 may be permanently or removably placed in the patient receiving space 2 and the patient accesses such space by simply walking therein and lies on such patient table 8. The patient table may have height adjustment means in the base and/or the support legs thereof. Several different constructions of patient table means or structures are known and envisageable, having a lifting or height adjustable support top. Furthermore, the patient table and particularly its support top 108 may have means for tilt adjustment of such top 108 along two longitudinal and/or transverse axes or along a single axis or by means of ball joints. Figure 7 shows a schematic example of a tilting support for the plate 108 of the patient table 8. The base part has an arched bracket 208 which is jointed at its top to a lower extension 308 of each lateral longitudinal edge of the patient table top 108 for the latter to be able to tilt about a horizontal axis. Removable locking means release the top so that it is free to tilt to a predetermined position, such removable locking means being then engaged to hold the patient table in the selected tilted position. The removable locking means may be of any type, such as transverse pins to be fitted in coincident holes in one of the extensions 308 of the bracket 208, the bracket having a succession of spaced holes formed therein coincident with the path of the hole of the lower extension 308 as the table top 108 is tilted.

In accordance with a further improvement or alternative arrangement, the patient table 8 may have a carriage-mounted base like the one designated by numeral 408 in Figure 7. In this case, the carriage-mounted base 408 may also have braking means for position locking.

The patient may access the patient receiving space by simply walking therein and only lie on the table when he/she is in such patient receiving space, or he/she may be placed on the table outside the patient receiving space 2 of the magnet structure and be carried into said space on the table 8.

Any other device, such as an armchair and/or a convertible armchair may be provided instead of the patient table.

Besides allowing the patient to assume various postures, the means for positioning and/or bearing and/or retaining and/or supporting the patient or for assisting placement thereof act at least partly as guides or cases for receiving several different devices or means, such as the resonance signal receiving means and the cables for connecting the outputs of such means to an electronic processor unit of the apparatus, associated to the magnet structure and located outside the latter.

According to the invention, the resonance signal receiving means may be removably fixed directly to the body under examination or a part thereof. These resonance signal receiving means, i.e. receiving coils are removably fixable directly to the patient body in the area of one or more separate anatomic regions to be imaged.

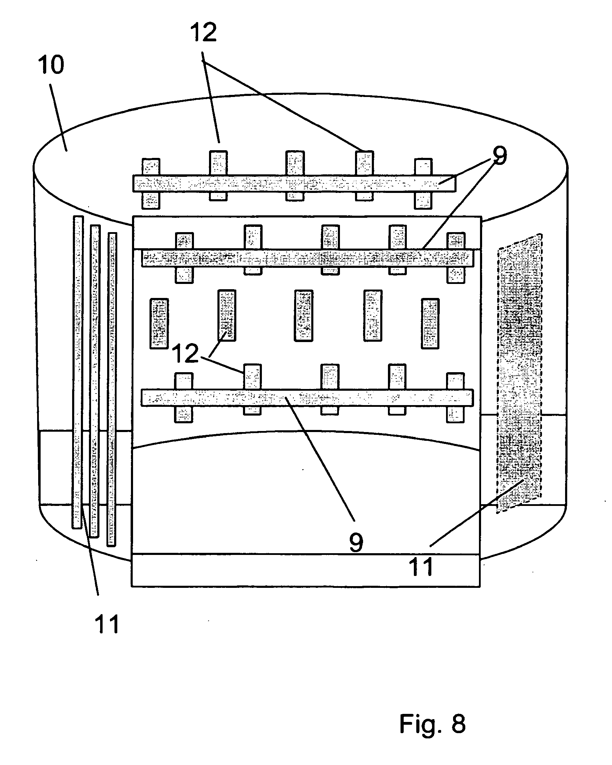

According to an advantageous feature of this invention, these receiving means comprise an electromagnetic (RF) signal receiving antenna, composed of conductors 9, which are removably fitted on a wearable support element. Referring to Figure 8, the wearable support element 10 is formed of a band, a strip or the like, which may be removably fastened around the body under examination or a part of such body under examination. Such strip, band or the like has two mutual connection ends, with means 11 for removably fixing and fastening the band or strip around a part of the body under examination, such as Velcro means and/or button-like means and/or other means such as laces or pairs of straps that can be connected together by interlocking end buckles, having strap pulling means. The wearable support element 10 may advantageously be at least partly or wholly made of an elastically extensible material.

The conductors 9 are coupled to the wearable support element 10 by removable fastener means, as shown in Figures 8 to 11 and designated by numeral 12. The above receiving means are not shown in detail for their being readily understandable without being illustrated.

The removable fastener means 12 may advantageously consist of Velcro strap elements. For a single wearable element 10 to be used for several different coils or receiving means having different structures or shapes and particularly formed of conductors that extend in different predetermined paths, the wearable element has a certain number of fastener elements 12 arranged on its surface in a predetermined pattern or order The fastener elements 12, in the form of connecting strap elements, cooperate with complementary elements of such connecting strap, that are attached to the rigid or flexible conductors.

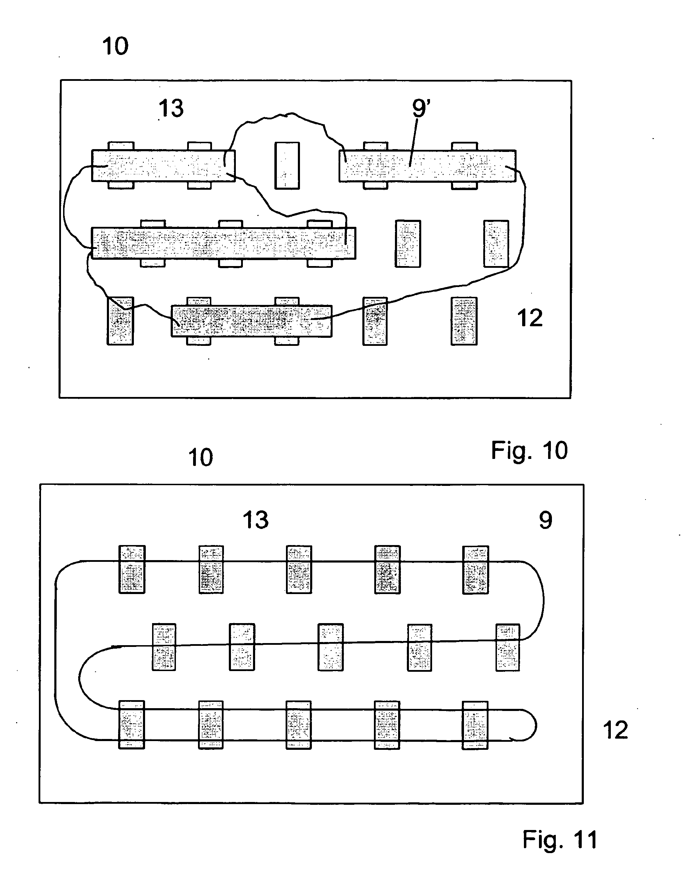

As mentioned above and shown in Figures 10 and 11, the receiving means, i.e. the antenna element, may be formed of a one-piece construction element designated by numeral 9 in Figure 11 (not an embodiment of the invention) and comprising rigid conductors which extend in a predetermined rigid three-dimensional path or, according to the invention, the receiving means are composed of several elastically or inelastically flexible conductors.

In order that the patient may be allowed to assume several different postures using a single receiving means, in addition to the use of elastically or inelastically flexible conductors, the invention provides, as shown in Figure 10, an antenna element, i.e. a receiving coil, composed of segments 9' of rigid and/or elastically or inelastically flexible conductors, which are connected together by removable rigid and/or elastically or inelastically flexible electric connection bridges.

The segments 9' of rigid or elastically or inelastically flexible conductors, that are connected together by removable electric connection bridges 13, which in turn can be rigid or elastically or inelastically flexible, have means for removable attachment to cooperating removable fastener elements 12 on the wearable support element 10, which removable fastener means 12 and which cooperating attachment means are respectively arranged along the segments 9' of rigid and/or elastically or inelastically flexible conductors and along the surface of the wearable support element 10. The above is provided in such a manner that, as each removable attachment means is coupled with each corresponding removable fastener element, the segments 9' of rigid or flexible conductors are automatically shaped and/or positioned relative to each other in a predetermined three-dimensional path pattern of said conductors to obtain the shape of the antenna or the receiving coil.

Therefore, the receiving coil is formed of a plurality of segments 9' of rigid or flexible conductors that may be removably attached to the wearable support element 10, and are electrically connected together by removable electric connection bridges 13, a set of such segments 9' of conductors being provided, which comprises pieces of different lengths and/or shapes for modular construction of conductors of different lengths and shapes, such as rectilinear segments of different lengths and/or curved segments having different lengths and/or different curvatures.

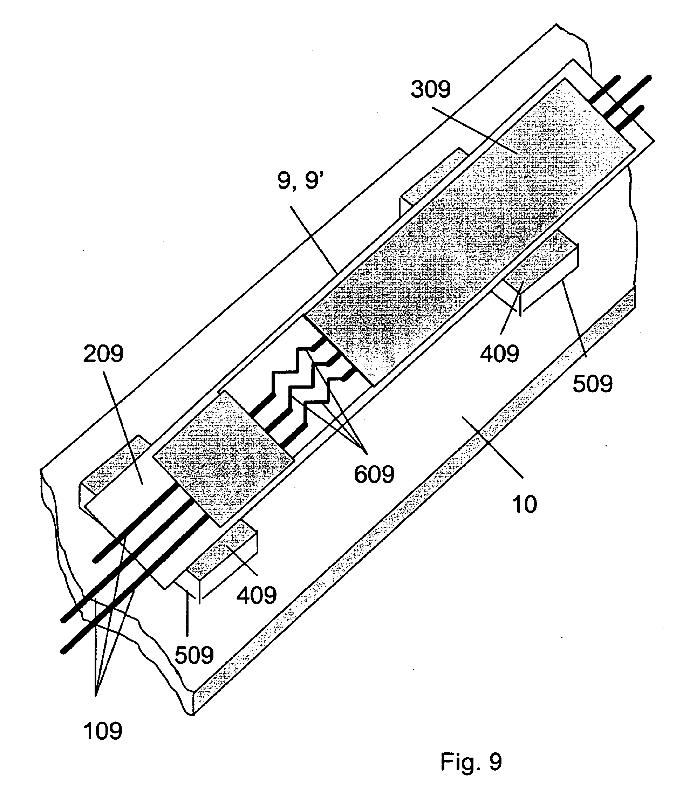

Figure 9 shows an enlarged detail of a possible embodiment of an elastically or inelastically flexible and/or extensible conductor 9, or a segment 9' of rigid or elastically or inelastically flexible conductor. In this case, one or more conductors consisting of a wire, a strap or a track of electrically conductive material 109 are held in a channel between two straps of flexible material, designated by numerals 209 and 309, which are fixed together, for instance glued and/or sewn and/or welded along their two lateral longitudinal edges. The strap 209 on the wearable garment side 10 consists of one of the two strap parts of a Velcro removable fastening device or has such strap part of the Velcro coupling device attached thereon all along its length or at parts thereof. These strap parts 409 engage with corresponding and complementary strap parts 509 which are fixed in coincident positions on the surface of the garment-like element 10.

According to a further feature allowing to use elastically or inelastically conductor segments 9' or conductors 9, the wires, straps or tracks 109 of conductive material have high-density portions arranged along part or all of their length, such as zigzag, wavy and/or spiral sections, or the like. These sections, designated by numeral 609 in Figure 9, include excess lengths of wire or the like. The provision that at least in such areas or all along their length, the wires 109 are held in an elastically extensible and/or deformable sheath that is formed, for instance, by the two straps 209 and 309, allows the conductor or a conductor segment 9, 9' to form a flexible and elastically extensible receiving coil, which can fit a variety of patient postures.

In a further variant, the attachment means 13, like the Velcro elements, are attached to the wearable element 10 using bridges of elastically deformable and/or extensible material.

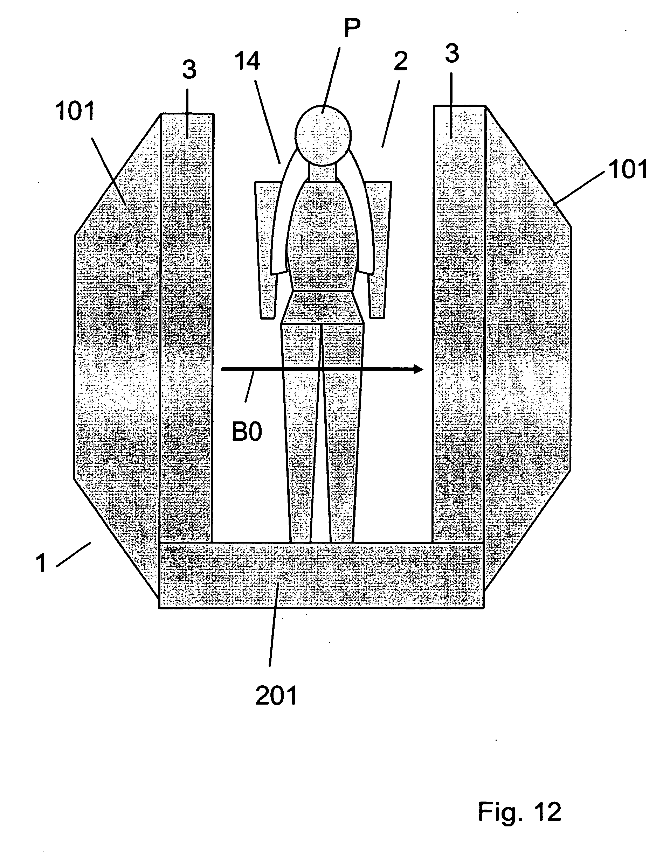

Particularly referring to the example of Figure 12, means may be provided, designated by numeral 14, which consist of a garment-like and/or wearable element containing one or more ballast elements. The ballast elements have a predetermined weight and may be permanently and/or removably connected to the garment element and/or replaced with other ballast elements of different weight.

Advantageously, the garment-like and/or wearable element is the same element as the one that is used for supporting the antenna or coil element, suitably having connections, pockets or the like for holding one or more ballast elements.

The method of use of the MRI apparatus is apparent from the above description. In the MRI imaging method with the apparatus of the invention, the patient
- accesses the imaging cavity, or the patient receiving space 2, by simply walking therein,
- autonomously assumes a position, or can autonomously assume a position as instructed by the operating personnel;
- is submitted to an imaging process.

For a better and more effective patient placement, shapes and/or positioning marks may be drawn, possibly in different colors, on the walking surface of the patient receiving space 2, here corresponding to the base 201 and/or on one or more of the walls of the magnet structure which delimit such patient receiving space 2, here corresponding to the vertical walls 101 and/or to the magnetic field generating means 3, such shapes being provided for use by the operating personnel as patient positioning examples. Therefore, in this case, the patient may be asked to place his/her feet in the feet positioning shapes on the floor of the space 2, which have a predetermined color, and to assume a posture as indicated by a body shape, a silhouette or the like, having a predetermined color or defined by an outline of a predetermined color on one or both vertical walls 101 or on the means 3.

Before entering the patient receiving space 2 and/or possibly simply before assuming the posture required for the examination and before being submitted to imaging, the patient has to wear one or more receiving coils as described above

When an examination requires means for assisting the patient in assuming a certain posture, then the examination includes the steps of:
- introducing a means for positioning and/or bearing and/or retaining and/or supporting a patient or for assisting placement thereof in the imaging space, in a predetermined position for the type of examination to be performed with reference to the anatomic region to be imaged;
- having the patient autonomously access the imaging space and autonomously sit or lie on such means for positioning and/or bearing and/or retaining and/or supporting the patient or for assisting placement thereof;
- so that the patient assumes the position or posture imposed by such means for positioning and/or bearing and/or retaining and/or supporting the patient or for assisting placement thereof;
- carrying out the MR imaging process;
- having the patient autonomously leave such means for positioning and/or bearing and/or retaining and/or supporting the patient or for assisting placement thereof;
- having the patient leave the imaging space by autonomous walking.

Alternatively, when the means for positioning and/or bearing and/or retaining and/or supporting the patient or for assisting placement thereof have a carriage-mounted base and when such means for positioning and/or bearing and/or retaining and/or supporting the patient or for assisting placement thereof can access the patient receiving space 2 through the access opening, then, as an alternative to the above, the patient sits or lies on said means for positioning and/or bearing and/or retaining and/or supporting the patient or for assisting placement thereof when such means are outside the patient receiving space as delimited by the magnet structure and then accesses such patient receiving space on such means for positioning and/or bearing and/or retaining and/or supporting the patient or for assisting placement thereof, driven or pushed by external means, which may be manual and patient-driven or motor-driven under the control of the patient or third parties.

As patient placement means a platform is provided on which the patient is asked to climb, which platform has an access side with one or more steps, the patient being positioned with both legs on a step or on the top of the platform or with one leg resting on the first or on one of the next steps, and the other leg resting on the base of the imaging space and/or on the top of the base.

Alternatively or additionally, as placement means, a chair element is provided, having a seating surface, a backrest and possibly a footrest, one or more of such seating, backrest and/or footrest surfaces being adjustable in position and/or tilt and/or orientation.

Once the patient is in the sitting position, either inside the patient receiving space or outside it, as provided by the above alternative methods, the seating surface and/or the backrest and/or the footrest may be adjusted in position and/or tilt or orientation.

Then, with the patient sitting and positioned within the patient receiving space, MR imaging is performed.

The position and/or tilt or orientation of the seating surface and/or the backrest and/or the footrest may be changed a first time and/or one or more additional times, and MR imaging may be repeated each time.

Once imaging is completed, the patient is either asked to stand up while still in the patient receiving space and to leave the space by autonomous walking, or carried out of the space on the chair, using the latter as a means of transport.

When using patient bearing and/or retaining means that may be set in various positions within the imaging space, the imaging method includes the steps of:
- positioning one or more of such retaining elements relative to the magnet and to a patient position, so that the patient assumes a predetermined position when he/she rests on such retaining means;
- having the patient access the imaging space by autonomous walking and autonomously assume the above mentioned position;
- carrying out the MR imaging process;
- possibly adjusting the position of one or more retaining elements or provide one or more further retaining elements in one or more further different positions within the imaging space and repeating the MR imaging process each time;
- having the patient stand up and leave the imaging space by autonomous walking.

Imaging may also be performed by one or more successive imaging steps, the patient being asked, before at least one of such steps or some of such steps, to wear a garment or a wearable and removably fastenable element, which acts as a ballast element 14.

A sequence of successive MR imaging operations may be performed, the weight of the ballast element being increased or decreased before each imaging operation, from a predetermined initial value and/or according to predetermined variation functions.

According to yet another possibility, the patient performs a predetermined movement during MR imaging, such as a walking and/or climbing movement, the base being provided in the form of a tilting treadmill and/or a step-up or step-down movement and/or a sitting movement or a passage from the sitting position to the standing position, or a forward bending movement or a backward and/or sideward arching movement, while a time-dependent sequence of MR imaging operations on at least one or more anatomic regions is performed during such movements.

## Claims

1. An MRI apparatus comprising a magnet structure which delimits a cavity in which a body under examination or a part thereof is received, and which includes means for generating a magnetic field in said cavity, as well as means for causing the body under examination or the part thereof to emit nuclear magnetic resonance signals and means for receiving said nuclear magnetic resonance signals,
the MRI apparatus further including an electronic processing unit to which the said means for receiving nuclear magnetic resonance signals are electrically connected;
the cavity having at least one access opening through which a patient can access said cavity by simply walking therein;
the said means for receiving nuclear magnetic resonance signals comprise a wearable support element which has means for being fastened removably to the body under examination at one anatomic region;
the said means for receiving nuclear magnetic resonance signals comprise an electromagnetic RF signal receiving antenna, the said electromagnetic RF signal receiving antenna is formed of a plurality of separated segments (9'), which segments are removably attached to the support element (10), and are electrically connected together by removable electric connection bridges (13);
the said segments (9') are formed by rigid or elastically or inelastically flexible conductors and have removable attachment means for removable attachment to cooperating removable fastener elements (12) provided on the wearable support element, which removable attachment means and which cooperating removable fastener elements are arranged respectively along said segments (9') of conductors and along the surface of the said support element in such a manner that each removable attachment means is coupled with each corresponding removable fastener element (12), the said segments (9') of rigid or elastically or inelastically flexible conductors are shaped and positioned relatively to each other to obtain the shape of the antenna.

2. An apparatus according to claim 1, in which the fastener elements (12) of the support element (10) and the attachment means of the segments (9') are Velcro strap elements.

## Patentansprüche

1. MRI-Gerät aufweisend
- einen Magnetrahmen, der einen Hohlraum begrenzt, in dem ein zu untersuchender Körper oder ein Teil davon aufgenommen wird, und der Mittel zur Erzeugung eines Magnetfelds in dem Hohlraum umfasst, und
- Mittel, die bewirken, dass der zu untersuchende Körper oder ein Teil davon nukleare magnetische Resonanzsignale aussendet, sowie Mittel zum Empfangen der nuklearen magnetischen Resonanzsignale,
wobei
- das MRI-Gerät weiterhin eine elektronische Verarbeitungseinheit umfasst, mit welcher die Mittel zum Empfangen der nuklearen magnetischen Resonanzsignale elektrisch verbunden sind,
- der Hohlraum wenigstens eine Zugangsöffnung hat, durch welche ein Patient hindurch tritt und somit in einfacher Weise in den Hohlraum gelangen kann,
- die Mittel zum Empfangen der nuklearen magnetischen Resonanzsignale ein tragbares Halterungsteil mit Mitteln zur abnehmbaren Anbringung an dem zu untersuchenden Körper in einem anatomischen Bereich aufweisen,
- die Mittel zum Empfangen von nuklearen magnetischen Resonanzsignalen eine elektromagnetische Empfangsantenne für HF-Signale aufweisen, die aus mehreren separaten Segmenten (9') besteht, die an dem Halterungsteil (10) lösbar befestigt sind und durch abnehmbare elektrische Verbindungsbrücken (13) elektrisch miteinander verbunden sind,
- wobei die Segmente (9') aus starren oder aus elastischen oder nichtelastischen flexiblen Leitern bestehen und lösbare Anbringungsmittel zur lösbaren Anbringung an zusammenwirkenden lösbaren Befestigungselementen (12), die an dem tragbaren Halterungsteil vorgesehen sind, aufweisen, wobei die lösbaren Anbringungsmittel und die zusammenwirkenden lösbaren Befestigungsmittel jeweils entlang der Segmente (9') der Leiter und entlang der Oberfläche des Halterungsteils in einer solchen Weise angeordnet sind, dass jedes lösbare Anbringungsmittel jeweils mit einem entsprechenden lösbaren Befestigungselement (12) gekoppelt ist, und wobei die Segmente (9') aus starren oder aus elastischen oder nichtelastischen flexiblen Leitern so gestaltet und relativ zueinander positioniert sind, um die Form der Antenne zu erhalten.

2. Gerät nach Anspruch 1, bei dem die Befestigungselemente (12) des Halterungsteils (10) und die Anbringungsmittel der Segmente (9') Klettverschlusselemente sind.

## Revendications

1. Dispositif IRM comprenant une structure d'aimants qui délimite une cavité dans laquelle un corps en cours d'examen ou une partie de celui-ci est reçu, et qui comprend des moyens pour générer un champ magnétique dans ladite cavité, ainsi que des moyens pour amener le corps en cours d'examen ou la partie de celui-ci à émettre des signaux de résonance magnétique nucléaire et des moyens pour recevoir lesdits signaux de résonance magnétique nucléaire,
le dispositif IRM comprenant en outre une unité de traitement électronique à laquelle lesdits moyens pour recevoir des signaux de résonance magnétique nucléaire sont connectés électriquement ;
la cavité comportant au moins une ouverture d'accès à travers laquelle un patient peut accéder à ladite cavité simplement en marchant dans celle-ci ;
lesdits moyens pour recevoir des signaux de résonance magnétique nucléaire comprennent un élément de support portable qui comporte des moyens destinés à être attachés de manière amovible au corps en cours d'examen au niveau d'une région anatomique ;
lesdits moyens pour recevoir des signaux de résonance magnétique nucléaire comprennent une antenne de réception de signal électromagnétique RF, ladite antenne de réception de signal électromagnétique RF est constituée d'une pluralité de segments (9') séparés, lesquels segments sont fixés de manière amovible à l'élément de support (10), et sont connectés électriquement les uns aux autres par des ponts de connexion électrique (13) amovibles ;
lesdits segments (9') sont formés par des conducteurs rigides ou flexibles élastiquement ou non élastiquement et comportent des moyens de fixation amovibles pour une fixation de manière amovible à des éléments d'attache (12) amovibles coopérants prévus sur l'élément de support portable, lesquels moyens de fixation amovibles et lesquels éléments d'attache amovibles coopérants sont agencés, respectivement, le long desdits segments (9') des conducteurs et le long de la surface dudit élément de support de manière à ce que chacun des moyens de fixation amovibles soit couplé à chaque élément d'attache (12) amovible correspondant, lesdits segments (9') de conducteurs rigides ou flexibles élastiquement ou non élastiquement sont formés et positionnés les uns par rapport aux autres pour obtenir la forme de l'antenne.

2. Dispositif selon la revendication 1, dans lequel les éléments d'attache (12) de l'élément de support (10) et les moyens de fixation des segments (9') sont des éléments de bandes Velcro.
